Europäisches Patentamt

European Patent Office

Office européen des brevets

(19)

(11) Veröffentlichungsnummer: **0 180 925**
**B1**

(12) # EUROPÄISCHE PATENTSCHRIFT

(45) Veröffentlichungstag der Patentschrift:
07.01.88

(21) Anmeldenummer: 85113900.6

(22) Anmeldetag: 31.10.85

(51) Int. Cl.⁴: **C 07 C 21/06,** C 07 C 17/34,
C 07 C 17/38

(54) Verfahren zur Herstellung von Vinylchlorid durch thermische Spaltung von gereingtem 1,2-Dichlorethan.

(30) Priorität: 07.11.84 DE 3440685

(43) Veröffentlichungstag der Anmeldung:
14.05.86 Patentblatt 86/20

(45) Bekanntmachung des Hinweises auf die Patenterteilung:
07.01.88 Patentblatt 88/1

(84) Benannte Vertragsstaaten:
BE DE FR GB IT NL SE

(56) Entgegenhaltungen:
DE - A - 3 024 156
US - A - 4 324 932

(73) Patentinhaber: WACKER-CHEMIE GMBH,
Prinzregentenstrasse 22, D-8000 München 22 (DE)

(72) Erfinder: Dummer, Gerhard, Dipl.-Ing. (TU),
Lohnerstrasse 12, D-8269 Burgkirchen (DE)
Erfinder: Haselwarter, Klaus, Dipl.-Ing. (FH),
Hauptstrasse 15 b, D-8261 Emmerting (DE)
Erfinder: Klaus, Hermann, Dipl.-Ing. (FH),
Lindenstrasse 4, D-8261 Marktl (DE)
Erfinder: Schmidhammer, Ludwig, Dr. Dipl.-Chem.,
Pappelweg 5, D-8261 Halming (DE)
Erfinder: Strasser, Rudolf, Dr. Dipl.-Chem.,
Lindacherstrasse 58, D-8263 Burghausen (DE)

## Beschreibung

Bekanntlich wird Vinylchlorid (VCM = Vinylchloridmonomer) grosstechnisch durch Spaltung von gereinigtem 1,2-Dichlorethan (EDC) hergestellt, die jedoch nicht quantitativ verläuft. Dabei wird das 1,2-Dichlorethan in Dampfform in einem Pyrolyseofen indirekt erhitzt und bei Temperaturen von 480°C bis 540°C unter einem Druck von 10-36 bar absolut zu Vinylchlorid und Chlorwasserstoff (HCl) gespalten, wobei durch entsprechende Temperaturregulierung im Spaltofen Umsetzungsgrade von üblicherweise 50 bis 60%, bezogen auf den Gesamtdurchsatz an dampfförmigem 1,2-Dichlorethan, eingehalten werden.

Die Verdampfung von EDC kann in einem separaten, externen Verdampfer mit Hilfe von Heizdampf entsprechenden Druckes oder aber auch — vor allem bei Drücken grösser 15 bar absolut — im Oberteil des Spaltofens durch Wärmetausch mit den bei der Spaltofenbefeuerung anfallenden Rauchgasen erfolgen, da für erhöhten Systemdruck entsprechend hochgespannter Heizdampf insbesondere aus Wirtschaftlichkeitsgründen (Stromerzeugung) üblicherweise nicht zur Verfügung steht.

Klammert man den Energiebedarf für die Verdampfung von EDC selbst also aus, so sind für die Befeuerung eines Spaltofens je 100 kg zu erzeugendes VCM etwa 0,3 Giga-Joule (GJ) an Wärmeenergie aufzubringen. Rund 85% dieser Wärmemenge werden für die Vorwärmung von flüssigem EDC auf nahezu Siedetemperatur, für die Überhitzung von dampfförmigem EDC auf Spalttemperatur sowie für den endothermen Spaltprozess benötigt, 25% davon gehen jedoch mit den Rauchgasen der Spaltofenbefeuerung verloren, da eine Wärmerückgewinnung wegen des dabei sich einstellenden, relativ niedrigen Rauchgastemperaturniveaus von etwa 270°C bis 330°C kaum wirtschaftlich ist.

Zwar sind einer wirtschaftlichen Notwendigkeit in der heutigen Zeit folgend in jüngster Zeit Verfahren vorgeschlagen worden, einen Teil der für die Spaltung von EDC erforderlichen Wärmemenge — soweit sie als Wärmeinhalt der Spaltgase auftritt — zurückzugewinnen, und die Rückgewinnung von Abwärme aus den Rauchgasen einer Spaltofenbefeuerung unter Erzeugung von Dampf wird in einigen Fällen ebenfalls praktiziert, wegen der relativ niedrigen Rauchgastemperatur sind derartige Verfahren jedoch wenig wirtschaftlich.

Ein Ziel der Erfindung ist es daher, durch Kombination mit einer anderen Massnahme die Rauchgastemperatur so anzuheben, dass die Wärmerückgewinnung wirtschaftlich vertretbar ist und den technologischen Aufwand rechtfertigt, ohne jedoch dabei Energieverluste zu erhöhen.

Gereinigtes EDC als Einsatzstoff für die Vinylchloriderzeugung gewinnt man durch Destillation von rohem EDC aus der Direkt- und/oder Oxichlorierung von Ethylen in zwei hintereinander geschalteten Kolonnen zur gemeinsamen Abtrennung von Wasser und Leichtsiedern bzw. von Hochsiedern. Da aber auch das nicht umgesetzte EDC aus der EDC-Spaltung vor seinem Wiedereinsatz im Spaltofen zumindest von Hochsiedern, die während des Spaltprozesses entstanden sind bzw. sich aus Leichtsiedern durch reaktive Umwandlung in Hochsiedern im Spaltofen gebildet haben, befreit werden muss, ist naturgemäss die meiste Wärmeenergie zur Kolonnenbeheizung der Hochsiederkolonne aufzuwenden. Je nach Spaltungsgrad im Spaltofen setzt sich somit das Zulaufgemisch zur Hochsiederkolonne aus etwa 50 bis 60% an entwässertem und von Leichtsiedern befreitem EDC als Sumpfprodukt der vorgeschalteten Leichtsiederkolonne und aus etwa 40 bis 50% an nicht umgesetztem EDC aus dem Spaltprozess zusammen, welches zurückgeführt worden ist. Die Leichtsiederkolonne hingegen verarbeitet vielfach nur rohes EDC aus der Synthese durch Direkt- und/oder Oxichlorierung von Ethylen.

Derartige Destillations- bzw. Rektifikationskolonnen, insbesondere die zur Hochsiederabtrennung, werden normalerweise überwiegend am Kolonnensumpf (an der Blase) mit Wasserdampf beheizt. Am Kopf einer solchen Kolonne wird die leichter siedende Fraktion abgezogen, kondensiert und zur Einhaltung eines bestimmten Rückflussverhältnisses als Flüssigkeit wieder am Kopf teilweise eingespeist. Der andere Teil des Kondensats wird als flüssiges Reinprodukt entnommen. Aus der Blase, in der je nach Konzentration der Hochsieder und je nach Druckverlust über die Gesamthöhe der Kolonne eindeutig höhere Temperaturen als am Kolonnenkopf herrschen, wird das Sumpfprodukt abgezogen. Die Einspeisung des zur rektifizierenden Gemisches erfolgt üblicherweise in die Kolonne oberhalb des Kolonnensumpfes.

Der Reinigungsprozess ist sehr energieaufwendig, und es stellte sich daher als weitere Aufgabe der Erfindung, den enormen Wärmebedarf für die Hochsiederabtrennung aus 1,2-Dichlorethan (EDC), der bisher bei etwa 0,25 t Dampf je Tonne gereinigtes EDC bzw. bei etwa 0,65 t Dampf je Tonne erzeugtes VCM liegt, durch eine verbesserte Prozessführung drastisch zu reduzieren.

Gelöst werden diese Aufgaben durch ein Verfahren zur Herstellung von Vinylchlorid durch thermische Spaltung von gereinigtem 1,2-Dichlorethan bei Temperaturen von 480°C bis 540°C unter einem Druck von 10 bis 36 bar absolut unter teilweiser Ausnützung des Wärmeinhaltes der Rauchgase der Spaltofenbefeuerung zur Vorwärmung von flüssigem 1,2-Dichlorethan auf nahezu Siedetemperatur, unter Ausnützung der Rauchgasabwärme zur Dampfgewinnung, Kühlen des Spaltgasgemisches in mehreren Stufen und Abtrennung des Chlorwasserstoffes aus dem Spaltgasgemisch in einer sog. HCl-Kolonne sowie Abtrennung von Vinylchlorid aus dem Spaltgasgemisch in einer sog. VCM-Kolonne, dadurch gekennzeichnet, dass man die Beheizung der Hochsiederkolonne zur Reinigung des 1,2-Dichlorethans, die mit einem Kopfdruck von 2 bis 3,1 bar absolut betrieben wird, durch Wärmetausch zwischen dem Sumpfprodukt und den auf 3,2 bis 6,2 bar absolut verdichteten Brüden bei einer Tem-

peraturdifferenz von 8 bis 22°C vornimmt, die Sumpftemperatur durch entsprechenden Blasenabzug bei 120°C bis 135°C hält, das durch entsprechend den erhöhten Temperaturen erniedrigte Verdampfungswärmen sich ergebende Wärmedefizit auf der wärmeabgebenden Seite des Wärmetauschers durch Entspannung entsprechender Mengen an Rücklauf kompensiert, den Teil des Kondensates aus dem Sumpfwärmetauscher, der als Rücklauf in die Kolonne zurückgeführt wird, im Wärmetausch mit Blasenprodukt aus der HCl-Kolonne, das zur VCM-Kolonne fliesst, auf die dem jeweiligen Kopfdruck entsprechende Sattdampftemperatur abkühlt, wobei sich das Blasenprodukt der HCl-Kolonne entsprechend anwärmt und sogar teilweise verdampft, den Rest des im Wärmetauscher anfallenden Brüdenkondensates, welches das gereinigte 1,2-Dichlorethan darstellt, ungekühlt mit einer Temperatur von 125°C bis 155°C direkt in den Economizer des thermischen Spaltofens pumpt und aus den Rauchgasen der Spaltofenbefeuerung bei einem Temperaturniveau von 350°C bis 550°C in einem Abhitzekessel durch Wärmetausch mit siedendem Wasser Mitteldruckdampf erzeugt, wobei das Kesselspeisewasser für den Abhitzekessel in einem dem Abhitzekessel nachgeschalteten Wärmetauscher durch Wärmetausch mit den Rauchgasen bei einem entsprechend niedrigerem Temperaturniveau von 60°C bis 120°C auf Siedetemperatur im Abhitzekessel vorgewärmt wird.

Überraschenderweise kann trotz eines ungünstigen Adiabatenexponenten das Kopfprodukt der Hochsiederkolonne (der Brüden), das fast ausschliesslich EDC enthält, soweit technisch möglich, adiabatisch verdichtet werden, ohne dass eine Kondensation des Brüden auftritt. Dadurch ist es möglich, praktisch die gesamte Kondensationswärme des Brüden zur Erhitzung der Hochsiederkolonne zu verwenden.

Überraschend war auch, dass der Brüden, bestehend aus dem gereinigten EDC, das noch Verunreinigungen von bis ca. 1 Gew.-% enthält, problemlos bis zu mindestens 15 bar aus seinem Sattdampfzustand verdichtet werden kann, ohne dass es zu den erwähnten Schwierigkeiten einer eventuellen vorzeitigen Kondensation kommt. Auch treten überraschenderweise keine Schwierigkeiten dadurch auf, dass die Verunreinigungen bzw. das EDC selbst sich beim Verdichten zersetzen könnten, zum Beispiel zu korrosiv wirkenden Substanzen oder polymerartigen oder teerigen Crackprodukten.

Derartige Harzablagerungen könnten nämlich bei Schraubenverdichtern ein Anlaufen der Rotoren bzw. bei Turboverdichtern Verschleisserscheinungen durch Unwuchtigwerden sowie ganz allgemein Probleme der Wellenabdichtung verursachen. Bekanntlich kann nämlich 1,2-Dichlorethan gerade an heissen Metallflächen eine thermische, durch Chlorwasserstoff katalysierte Polykondensation unter Abspaltung weiteren Chlorwasserstoffs (Autokatalyse) und unter Ausbildung teerartiger Polymerisate erleiden.

Daneben enthält das sog. gereinigte EDC neben Chlorwasserstoff (HCl) auch noch andere Chlorkohlenwasserstoffe, die bei der thermischen Spaltung von EDC als Promotoren teils erwünscht sind bzw. teils aus Wirtschaftlichkeitsgründen in gewissen Grenzen zugelassen werden, weil sie erfahrungsgemäss keinen schädigenden Einfluss auf den Spaltprozess ausüben. Derartige Verunreinigungen, die im Bereich von je 0,01 bis 0,15 Gew.-% im gereinigten EDC-Dampf enthalten sind, sind z.B. Tetrachlorkohlenstoff, Chloroform, 1,1-Dichlorethan, 1,1,2-Trichlorethan, Trichlorethylen und isomere Dichlorethylen. Da diese genannten Verbindungen teilweise weit weniger thermisch stabil sind als 1,2-Dichlorethan, konnte man davon ausgehen, dass diese Verunreinigungen bei der Verdichtung unter Ausbildung von Harzprodukten grösstenteils zersetzt werden. Daneben war die Bildung äusserst korrosiver Zersetzungsprodukte aus einigen dieser Verunreinigungen zu erwarten.

So ist es bekannt, dass Tetrachlorkohlenstoff relativ leicht in Chlor- und Trichlormethylradikale gespalten wird, die hoch korrosiv sind und somit grosse Verdichterschäden verursachen können. Es war auch zu erwarten, dass durch mögliche Zersetzungsreaktionen sich aber beispielsweise das 1,2-Dichlorethan in seiner Zusammensetzung so entscheidend ändert, dass beim anschliessenden Spaltprozess u. U. Schwierigkeiten hinsichtlich Reinheit des erzeugten Vinylchlorids sowie Probleme bei Kinetik des Spaltprozesses auftreten.

Die Thermokompression (Brüdenverdichtung) ist umso wirtschaftlicher, je kleiner das Verdichtungsverhältnis der Kopfbrüden unter dem Aspekt einer genügend grossen Temperaturdifferenz zwischen verdichtetem Brüdendampf und Sumpftemperatur der Kolonne gehalten werden kann. Eine Temperaturdifferenz von 8 bis 22°C ist dabei ausreichend, um genügend Triebkraft für den Wärmetausch zwischen den verdichteten Brüden und dem Sumpfprodukt der Hochsiederkolonne bei wirtschaftlich vertretbaren Wärmetauschflächen zur Verfügung zu haben.

Andererseits kann der Kopfdruck der Kolonne nicht beliebig angehoben werden, da sonst die Blasentemperatur entsprechend dem erhöhten Blasendruck, der sich aus dem Kopfdruck und dem Druckverlust über die gesamte Kolonnenhöhe zusammensetzt, stark erhöht wird und damit auch die Sumpftemperatur ansteigt. Hierdurch kann ein schnelleres Fouling (schnellere Verunreinigung bis hin zum Zusetzen) des Wärmetauschers auf der Sumpfproduktseite eintreten. Die Praxis hat gezeigt, dass eine Sumpftemperatur von 120°C bis 135°C hinsichtlich Verschmutzung des Wärmetauschers vorteilhaft ist. Bei einem Druckverlust von 0,5 bar über die Kolonne und unter Wahrung der erforderlichen Temperaturdifferenz zwischen Brüdendampf und Sumpftemperatur ist es daher zweckmässig, die Brüden durch adiabate Kompression auf ein Temperaturniveau von 128°C bis max. 157°C zu bringen. Dies bedeutet Kolonnenkopfdrücke von 2 bar absolut bis max. 3,1 bar absolut bzw. Brüdendrücke nach der Verdichtung von 3,2 bar absolut bis max. 6,2 bar absolut. Das Verdichtungsverhältnis liegt demnach vorzugsweise bei Werten zwischen 1,6 und 2,0.

Um den Wärmeübergang zu verbessern, entfernt man vorzugsweise die geringe adiabate Überhitzungswärme durch Einspritzen von entsprechenden Mengen an flüssigem 1,2-Dichlorethan in die komprimierten Brüden vor Eintritt in den Wärmetauscher zur Kolonnenbeheizung. Das geringe Wärmedefizit, das sich aus den unterschiedlichen Verdampfungs- bzw. Kondensationswärmen auf der wärmeabgebenden Brüdenseite bzw. der wärmeaufnehmenden Kolonnensumpfseite des Wärmetauschers durch mit steigender Temperatur abfallende spezifische Verdampfungs- bzw. Kondensationswärmen ergibt, kann man vorzugsweise durch Entspannen unter Flashverdampfen eines entsprechenden Anteils an flüssigem Rücklauf kompensieren. Da der Rücklauf vor Eintritt in die Kolonne auf die Sattdampftemperatur der verdichtersaugseitigen Brüden gebracht werden muss, was durch entsprechendes Kühlen mit Wasser oder vorzugsweise durch Wärmetausch mit Sumpfprodukt aus der HCl-Kolonne unter Ausnützung des Wärmeinhaltes des Rücklaufes, der sich durch die Temperaturdifferenz der Brüden vor bzw. nach deren Verdichtung ergibt, geschieht, kann man aber auch den Rücklauf nur so weit abkühlen, dass bei Entspannung der gesamten Rücklaufmenge die nötige Flashdampfmenge entsteht und die dem Kolonnenkopfdruck entsprechende Sattdampftemperatur erreicht wird.

Die im Sumpfwärmetauscher anfallenden kondensierten Brüden haben somit eine Temperatur von etwa 125°C bis 155°C.

Pumpt man dieses Kondensat bzw. gereinigte 1,2-Dichlorethan ohne Zwischenkühlung direkt in den Economizer des Spaltofens, so steigt die Temperatur der Rauchgase der Spaltofenbefeuerung am Austritt aus dem Economizer auf 370°C bis 580°C an. Dies ist ein Temperaturniveau, bei dem eine Wärmerückgewinnung unter Dampferzeugung äusserst wirtschaftlich ist.

Fig. 1 zeigt eine vorteilhafte Ausführungsform einer Vorrichtung zur Durchführung des erfindungsgemässen Verfahrens.

Fig. 2 zeigt eine Versuchsvorrichtung für die Heizung der Hochsiederkolonne mittels Wärmetauscher und für die Untersuchung des Einflusses der Brüdenverdichtung auf die EDC-Zusammensetzung.

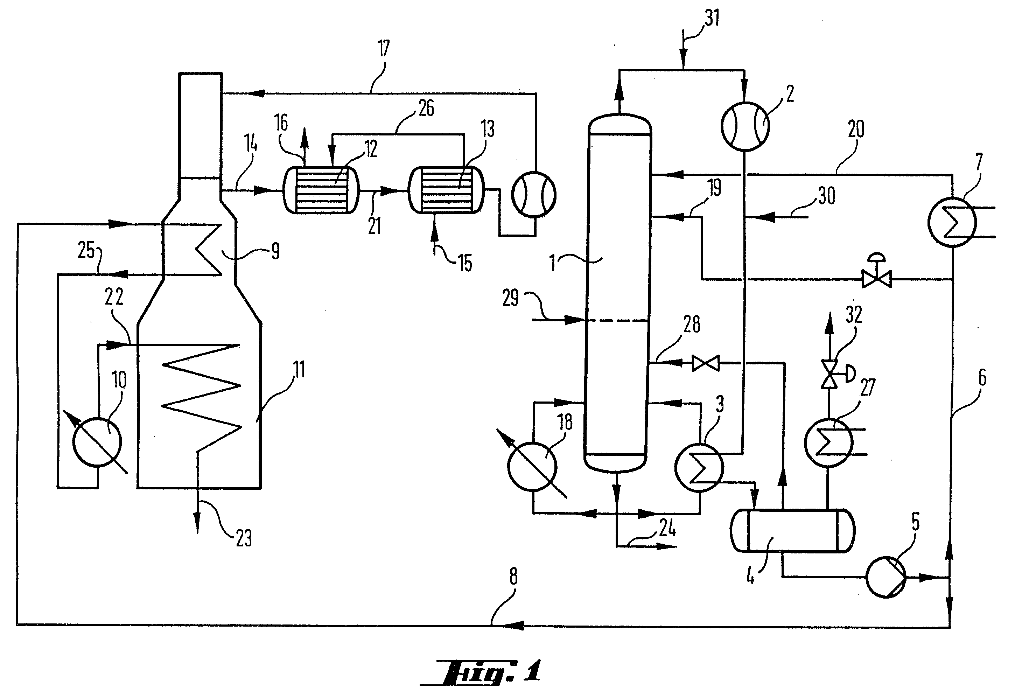

In die Hochsiederkolonne 1 von Fig. 1 wird bei 29 das aufzutrennende rohe EDC eingespeist. Die Kolonne 1 besitzt an ihrem Fussende, der Blase bzw. dem Kolonnensumpf zumindestens eine Abzugsvorrichtung, die zum einen die Möglichkeit bietet, die Hochsiederfraktion über die absperrbare Rohrleitung 24 abzuziehen, sowie über Rohrleitungen mit dem Sumpf Wärmetauscher 3 und gegebenenfalls mit einer zusätzlichen Heizungsvorrichtung 18 verbunden ist. Der Wärmetauscher sowie gegebenenfalls die Zusatzheizungsvorrichtung sind über weitere Rohrleitungen zur Rückführung des erhitzten Sumpfinhaltes in den unteren Teil der Kolonne verbunden.

Der Kolonnenkopf ist über Leitung mit einem Verdichter 2, z.B. einem Turboverdichter verbunden. Über die Einspeisung 31 kann zur Druckhaltung z.B. ein Inertgas wie Stickstoff eingespeist werden.

Die Druckseite des Verdichters 2 ist über Rohrleitung mit dem Sumpfwärmetauscher 3 und weiter mit dem Auffangbehälter 4 für das gereinigte kondensierte EDC verbunden. Bei 30 kann z.B. aus dem Behälter 4 EDC zusätzlich in die Verbindungsleitung vom Verdichter 2 zum Wärmetauscher 3 eingespeist werden, z.B. um die Temperatur des in 3 als Heizmedium dienenden EDC zu regeln oder zu steuern. Die gegebenenfalls vorhandene Zusatzheizung 18 sowie die absperrbare Leitung vom Behälter 4 zur Einspeisungsstelle 28 dienen üblicherweise lediglich zum Einfahren der Anlage, bis ein annähernd stationärer Zustand erreicht ist. Bei der üblichen Betriebstemperatur können nicht kondensierbare gasförmige Stoffe über den Kondensator 27, z.B. einen Wasserkühler, und das Regelventil 32 ausgeschleust werden. Die kondensierte EDC-Fraktion wird aus dem Behälter 4 über die Pumpe 5 abgezogen.

Ein Teil der EDC-Fraktion wird über die Rohrleitung 6 und den Wärmetauscher 7 sowie die Rohrleitung 20 wieder in die Verstärkersäule der Kolonne 1 eingespeist. Darüber hinaus besteht zur Ausregelung von Störungen bzw. als zusätzliche Wärmezufuhr die Möglichkeit, über eine absperrbare bzw. in ihrem Durchfluss regelbare Rohrleitung bei 19 weiteres ungekühltes EDC in die Verstärkerkolonne einzuspeisen.

Über die Rohrleitung 8 schliesslich kann das EDC direkt in den Economizer 9 des Spaltofens 11, von dort über eine Leitung 25 und den Verdampfer 10 bei 22 in den Spaltofen 11 eingeführt werden. Das Reaktionsgemisch wird bei 23 aus dem Spaltofen 11 abgezogen und (nicht dargestellt) der Aufarbeitung zugeführt.

Die Rauchgasleitung 14 leitet die Rauchgase über den Abhitzekessel 12, die Leitung 21 und Wärmetauscher 13 zu einem Gebläse, das die Rauchgase über Rohrleitung 17 ins Freie befördert. Bei 15 wird Wasser in den Wärmetauscher 13 eingeführt und erhitzt, dann über Rohrleitung 26 in den Abhitzekessel 12 geleitet, aus dem bei 16 der erzeugte Dampf abgeführt werden kann.

Durch das erfindungsgemässe Verfahren werden pro Tonne erzeugtes Vinylchlorid rund 0,65 to Dampf eingespart. Die für die Brüdenthermokompression benötigte elektrische Energie beträgt etwa 31 kWh/t erzeugtes Vinylchlorid, also nur rund 9% bezogen auf die Dampfersparnis bei rein kalorischer Betrachtung. Daneben werden aus der Rauchgasabwärme pro Tonne erzeugtes Vinylchlorid etwa 0,15 t Dampf (mit 16 bar) erzeugt, der anderweitig verwendet werden kann. Damit ergibt sich gemäss der Erfindung eine äusserst wirtschaftliche Vinylchloriderzeugung, da der Vinylchloridherstellungspreis durch Energieeinsparung bei der Hochsiederabtrennung von 1,2-Dichlorethan als Einsatzstoff für die Vinylchloridherstellung bzw. bei der VCM-Kolonne durch Wärmetausch von Rücklauf mit Blasenprodukt

aus der HCl-Kolonne und durch die Dampfgewinnung aus der Rauchgaswärmerückgewinnung mit rund 0,80 t/t Vinylchlorid weniger Dampf belastet wird. Trotz erhöhter Sumpftemperaturen in der Hochsiederkolonne beträgt die Standzeit des Sumpfwärmetauschers für die Brüdenbeheizung der Hochsiederkolonne rund ein Jahr.

Gemäss einer anderen Ausführungsform kann die Zusatzheizung 18 auch im annähernd stationären bis stationären Betrieb weiter benutzt werden, wenn man auf die Flashverdampfung durch Rückführung von gereinigtem EDC verzichten will.

Zwar ist auch denkbar, insbesondere bei hoher Verdichtung (z.B. bis 15 bar) im Turboverdichter 2 das gereinigte EDC aus dem Verdichter kommend direkt bei 22 in den Spaltofen 11 einzuspeisen, diese Ausführungsform stellt jedoch eine äusserst starre Verbindung von EDC-Reinigung und EDC-Spaltung dar, so dass bei Störungen entweder der Reinigung oder der Spaltung stets die gesamte Anlage darunter leidet und gegebenenfalls abgeschaltet werden muss. Demgegenüber bietet die erfindungsgemässe Verfahrensweise auch im Falle solcher Störungen die Möglichkeit, durch Zuschalten z.B. eines EDC-Tanklagers den jeweils ungestörten Teil der Anlage weitestgehend ungestört weiterzufahren. So kann z.B. entweder das frisch gereinigte EDC zwischengelagert werden oder bei Störung der EDC-Reinigung kann der Spaltofen z.B. aus einem solchen EDC-Tanklager weiterversorgt werden.

Es versteht sich, dass einzelne Merkmale der Vorrichtung bzw. des Verfahrens weggelassen werden können, ohne dass der allgemeine Erfindungsgedanke verlassen wird.

Die folgenden Beispiele dienen der weiteren Erläuterung der Erfindung. Alle Temperaturen verstehen sich in Celsiusgraden, alle Druckangaben als Absolutdrucke. Gew.-ppm bedeuten mg einer Substanz pro kg Gesamtgewicht der EDC-Fraktion.

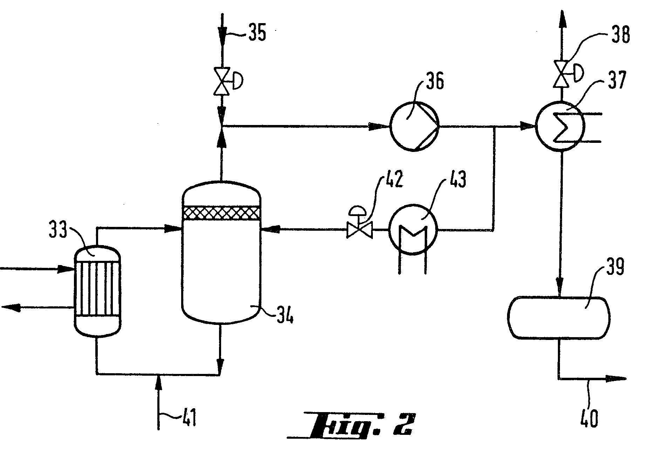

*Beispiel 1 (vgl. auch Figur 2):*

Es wurde 1,2-Dichlorethan folgender Zusammensetzung verwendet:

| 99,4 | Gew.-% | 1,2-Dichlorethan |
|---|---|---|
| 10 | Gew.-ppm | Vinylchlorid |
| 80 | Gew.-ppm | cis-1,2-Dichlorethylen |
| 60 | Gew.-ppm | trans-1,2-Dichlorethylen |
| 360 | Gew.-ppm | 1,1-Dichlorethan |
| 250 | Gew.-ppm | Chloroform |
| 1250 | Gew.-ppm | Tetrachlorkohlenstoff |
| 500 | Gew.-ppm | Trichlorethylen |
| 300 | Gew.-ppm | 1,1,2-Trichlorethan |
| 3000 | Gew.-ppm | Benzol |
| 150 | Gew.-ppm | Chlorwasserstoff |

8,32 t/h dieses 1,2-Dichlorethans wurden in einem mit Wasserdampf beheizten Rohrbündelumlaufverdampfer 33 bei einem Druck von 1,42 bar verdampft. Die zugehörige Sattdampftemperatur betrug 96°C. Das zu verdampfende 1,2-Dichlorethan wurde mit einem konstanten Strom über Leitung 41 zugeführt. Nach Trennung des aus dem Umlaufverdampfer 33 austretenden Flüssigkeitsdampfgemisches in einem mit einem Demister versehenen Abscheider 34 wurden die Brüden von einem einstufigen Schraubenverdichter 36 angesaugt. Zur Konstanthaltung des Saugdruckes während des Anfahrens bzw. zur Aufrechterhaltung des Enddruckes wurde bei 35 auf 97°C erhitzter Stickstoff zugespeist. Mit dem Verdichter 36 wurden die Brüden auf einen Druck von 5,05 bar komprimiert, wobei der Druck vom Enddruckregler 38 konstant gehalten wurde. Nach der Verdichtung erreichten die Brüden eine Temperatur von 158°C. Im wassergekühlten Kondensator 37 wurde der verdichtete 1,2-Dichlorethandampf kondensiert, das ablaufende Kondensat in einer Vorlage 39 gesammelt, von nicht kondensierbaren Bestandteilen (überwiegend Stickstoff) getrennt und über Leitung 40 zur Weiterverarbeitung an anderer Stelle entfernt. Die nicht kondensierbaren Bestandteile wurden über den Enddruckregler 38 ausgeschleust. Für das Anfahren und die Regelung des Schraubenverdichters 36 standen eine Bypassleitung mit Regelventil 42 und Kühler 43 zur Verfügung. Das bei 40 entfernte, kondensierte 1,2-Dichlorethan hatte nach der scharfen Behandlung in Dampfform, die durch adiabate Verdichtung mit etwa 10grädiger Überhitzung über die dem Enddruck entsprechende Sattdampftemperatur erfolgt war, folgende Zusammensetzung:

| 99,4 | Gew.-% | 1,2-Dichlorethan |
|---|---|---|
| 8 | Gew.-ppm | Vinylchlorid |
| 75 | Gew.-ppm | cis-1,2-Dichlorethan |
| 50 | Gew.-ppm | Trans-1,2-Dichlorethan |
| 361 | Gew.-ppm | 1,1-Dichlorethan |
| 249 | Gew.-ppm | Chloroform |
| 1252 | Gew.-ppm | Tetrachlorkohlenstoff |
| 500 | Gew.-ppm | Trichlorethylen |
| 302 | Gew.-ppm | 1,1,2-Trichlorethan |
| 2998 | Gew.-ppm | Benzol |
| 40 | Gew.-ppm | Chlorwasserstoff |

Bis auf eine geringfügige Abnahme der mit dem Stickstoff beim Enddruckregler 38 teilweise ausgeschleusten leichtersiedenden Bestandteilen des eingesetzten 1,2-Dichlorethans, wie Chlorwasserstoff, Vinylchlorid, trans-1,2-Dichlorethylen, cis-1,2-Dichlorethylen, war also kein Unterschied beim Vergleich der Analysen vor und nach der Verdichtung feststellbar. Nach 3 Wochen Versuchsbetrieb waren im Verdichter keine Ablagerungen und Verschmutzungen nachweisbar. Die Zusammensetzung des 1,2-Dichlorethans nach Brüdenverdichtung und Kondensation blieb immer die gleiche. Bei der Weiterverarbeitung des bei 40 ausgetragenen 1,2-Dichlorethans in Richtung Vinylchlorid ergaben sich keine Unterschiede zu nicht verdichtetem 1,2-Dichlorethan gleicher Zusammensetzung.

*Beispiel 2:*

Analog Beispiel 1 wurden dieselben Brüden mit einem zweistufigen Verdichter auf 15 bar verdichtet, wodurch sich die verdichteten Brüden auf 238°C erhitzten. Nach einer Woche Versuchsbetrieb im Durchlauf, wie unter Beispiel 1 beschrieben, erkannte man keine Unterschiede in der Zusammensetzung des 1,2-Dichlorethans vor bzw. nach der Verdichtung. Ablagerungen bzw. Verschmutzungen des Verdichters wurden ebenfalls nicht beobachtet.

*Beispiel 3:*

Es wurde analog Beispiel 1 vorgegangen mit dem Unterschied, dass das bei 40 anfallende kondensierte 1,2-Dichlorethan bei 41 wieder in den Verdampfer 33 eingeschleust wurde. Trotz des Kreisfahrens von 1,2-Dichlorethan änderte sich nach insgesamt 14 Tagen Versuchsbetrieb dessen Zusammensetzung nicht, ausgenommen jedoch die leichtsiedenden Verunreinigungen, die zwangsweise bei 38 mit dem Stickstoff ausgeschleust wurden. Das 1,2-Dichlorethan wurde unter Kreislaufführung etwa 670mal komprimiert.

*Beispiel 4:*

Es wurde analog Beispiel 1 vorgegangen. Die verdichteten Brüden wurden jedoch zur Verdampfung von 1,2-Dichlorethan verwendet, indem man den Wasserkühler 43 umging und die verdichteten Brüden anstelle des Heizdampfes in den Umlaufverdampfer 33 einleitete, wobei durch Wärmetausch der kondensierenden Brüden mit dem bei 41 mit Siedetemperatur zulaufenden 1,2-Dichlorethan die Verdampfung erfolgte. Die aus dem Umlaufverdampfer 33 ablaufenden kondensierten Brüden wurden über 37, 39 und 40 entfernt. Ein Teil des kondensierten Brüden wurde auf den Druck der Saugseite des Verdichters entspannt und in einer Menge von etwa 1 t/h der Saugseite des Verdichters 36 zugeführt, um die Wärmeverluste zu decken bzw. die Unterschiede der Verdampfungswärmen bei den unterschiedlichen Drücken und Temperaturen auszugleichen. Nicht kondensierter Stickstoff entwich bei 38.

Die Zusammensetzung des 1,2-Dichlorethans vor bzw. nach der Verdichtung blieb etwa die gleiche. Versuchsdauer: zwei Tage.

*Beispiel 5 (vgl. Figur 1):*

Die Hochsiederkolonne 1 wurde bei 29 mit 74,5 t/h rohem 1,2-Dichlorethan folgender Zusammensetzung beschickt:

| 99,08 | Gew.-% | 1,2-Dichlorethan |
|---|---|---|
| 10 | Gew.-ppm | Vinylchlorid |
| 80 | Gew.-ppm | cis-1,2-Dichlorethylen |
| 60 | Gew.-ppm | trans-1,2-Dichlorethylen |
| 360 | Gew.-ppm | 1,1-Dichlorethan |
| 250 | Gew.-ppm | Chloroform |
| 1250 | Gew.-ppm | Tetrachlorkohlenstoff |
| 480 | Gew.-ppm | Trichlorethylen |
| 3100 | Gew.-ppm | 1,1,2-Trichlorethan |
| 2980 | Gew.-ppm | Benzol |
| 160 | Gew.-ppm | Chlorwasserstoff |
| 350 | Gew.-ppm | andere Hochsieder |

Im Gleichgewichtszustand der Kolonne stellte sich durch kontinuierlichen Abzug von 1,5 t/h Sumpfprodukt bei 24 in der Blase der Hochsiederkolonne bei einem Druck von 3 bar eine Temperatur von 127°C ein. Am Kopf der Kolonne wurden bei einem Druck von 2,5 bar 136,4 t/h dampfförmiges Kopfprodukt mit einer Sattdampftemperatur von 118°C vom Turbokompressor 2 angesaugt und auf 4,5 bar verdichtet. Dabei erwärmten sich die Brüden auf 145°C, waren also mit ca. 3°C überhitzt. Die für die Brüdenverdichtung erforderliche elektrische Energie betrug 886 kWh. Bei 30 wurden etwa 1,7 t/h ca. 142grädiges flüssiges Kondensat aus dem Sammler 4 eingespritzt, um die Überhitzungswärme aus den Brüden wegzunehmen, wodurch der Wärmeübergang im Wärmetauscher 3 verbessert wurde. Der nun gesättigte Brüdendampf gelangte mit einer Temperatur von 142°C in den Wärmetauscher 3, der als Rohrbündelumlaufverdampfer mit Sumpfprodukt in den Rohren konzipiert war und wo die Brüden im Wärmetausch mit 127grädigem Sumpfprodukt auf der Mantelseite des Wärmetauschers kondensierten, während das umlaufende Sumpfprodukt in den Rohren durch die freiwerdende Kondensationswärme verdampfte und somit die Kolonne beheizte. Die kondensierten Brüden flossen aus dem Wärmetauscher 3 in den Sammler 4 ab.

Bei 32 wurde mittels eines Enddruckreglers der Verdichterdruck 4,5 bar gehalten. Nicht kondensierbare Bestandteile der verdichteten Brüden wurden nach Kühlung im Wasserkühler 27 über das Regelventil 32 ausgeschleust, insbesondere geringe Mengen Stickstoff, die zur Aufrechterhaltung des gewünschten Enddruckes bei 31 zugegeben wurden. Mit der Pumpe 5 wurde ca. 140grädiges Brüdenkondensat über Leitung 6 als Rücklauf in die Kolonne zurückgeleitet. Um das Wärmedefizit beim Wärmetauscher 3 auszugleichen, das dadurch entsteht, dass die spezifische Kondensationswärme von 1,2-Dichlorethan bei 142°C um rund 8,5 kJ/kg niedriger ist als die spezifische Verdampfungswärme von 1,2-Dichlorethan bei 127°C, und zur Deckung von Wärmeverlusten wurde über Leitung 19 soviel Rücklauf aus Leitung 6 in die Kolonne entspannt, dass ca. 4 t/h Entspannungsdampf anfielen. Der Rest des ca. 140grädigen Rücklaufs wurde über den Wärmetauscher 7 geleitet. Hier wurde der Rücklauf mit 81grädigem Sumpfprodukt aus der HCl-Kolonne, welches unter Entspannung von 9,8 bar auf etwa 6,5 bar zur nachfolgenden VCM-Kolonne strömte, vor Eintritt in die Kolonne über Leitung 20 auf 118°C gekühlt, während rund 16% des Sumpfproduktes der HCl-Kolonne verdampften. Dadurch wurden zur Beheizung der VCM-Kolonne nur 6,4 t/h 20 bar-Dampf benötigt.

73 t/h gereinigtes 1,2-Dichlorethan wurden mit einer Temperatur von etwa 140°C über Leitung 8 in den Economizer des Spaltofens bei 9 gepumpt. Das gereinigte 1,2-Dichlorethan hatte folgende Zusammensetzung:

| 99,4 | Gew.-% | 1,2-Dichlorethan |
|---|---|---|
| 11 | Gew.-ppm | Vinylchlorid |
| 78 | Gew.-ppm | cis-1,2-Dichlorethylen |
| 61 | Gew.-ppm | trans-1,2-Dichlorethylen |
| 359 | Gew.-ppm | 1,1-Dichlorethan |
| 250 | Gew.-ppm | Chloroform |
| 1250 | Gew.-ppm | Tetrachlorkohlenstoff |
| 481 | Gew.-ppm | Trichlorethylen |

298   Gew.-ppm   1,1,2-Trichlorethan
2981  Gew.-ppm   Benzol
158   Gew.-ppm   Chlorwasserstoff

Im Economizer 9 wurde das unter einem Pumpendruck von 17 bar stehende flüssige 1,2-Dichlorethan durch Wärmetausch mit den Rauchgasen der Spaltofenbefeuerung auf 190°C vorgewärmt. Anschliessend gelangte es über Leitung 25 in den mit Heizdampf beaufschlagten Verdampfer 10, wo es bei einem Druck von 13 bar entsprechend einer Siedetemperatur von etwa 195°C in den Spaltofen bei 22 verdampft wurde. Im Strahlungsteil des Spaltofens 11 wurde das dampfförmige 1,2-Dichlorethan zunächst auf Spalttemperatur erhitzt und dann bei einer Ofenaustrittstemperatur von etwa 507°C mit 61,2%igem Umsatz in Vinylchlorid und Chlorwasserstoff gespalten. Das Spaltgasgemisch, im wesentlichen aus 28 t/h Vinylchlorid, 16,4 t/h Chlorwasserstoff und 28,3 t/h nicht umgesetzten 1,2-Dichlorethan bestehend, verliess bei 23 den Spaltofen, wurde dann nach bekannten Methoden abgekühlt und gelangte teilweise gasförmig bzw. teilweise flüssig in die HCl-Kolonne, wo über Kopf 16,4 t/h Chlorwasserstoff abgezogen wurden. Das Sumpfprodukt der HCl-Kolonne, aus 28 t/h Vinylchlorid und 28,3 t/h nicht umgesetztem 1,2-Dichlorethan bestehend, wurde nach Erwärmung und teilweiser Verdampfung im Wärmetauscher 7 in der nachfolgenden VCM-Kolonne getrennt, wobei am Kopf 28 t/h reines Vinylchlorid anfielen, während über den Sumpfabzug 28,3 t/h nicht umgesetztes 1,2-Dichlorethan entfernt wurden, welches zur Reinigung für den erneuten Einsatz wieder zurückgeführt wurde.

Die Rauchgase, die bei der Spaltofenbefeuerung mit 2200 m³/h Erdgas und 25 300 m³/h Luft, die auf etwa 100°C angewärmt ist, anfielen, verliessen den Economizer 9 mit einer Temperatur von etwa 415°C und traten bei 14 in den Abhitzekessel 12 ein. Hier wurden 4,1 t/h Wasser mit nahezu Siedetemperatur, das über Leitung 26 in den Abhitzekessel eintrat, verdampft. Die 4,1 t/h 16 bar-Dampf wurden bei 16 ins Dampfnetz abgegeben. Am Austritt des Abhitzekessels 12 lag die Rauchgastemperatur bei etwa 210°C. Die Rauchgase strömten über Leitung 21 in den Kesselspeisewasservorwärmer 13, wo 4,1 t/h 80grädiges Kesselspeisewasser, welches bei 15 in den Vorwärmer eintrat, auf etwa 203°C vorgewärmt wurden, bevor es in den Abhitzekessel 12 über Leitung 26 eintrat. Die Rauchgase kühlten sich dabei im Vorwärmer 13 weiter ab und wurden mit Hilfe eines Saugzuggebläses über Leitung 17 mit einer Temperatur von etwa 146°C in die Atmosphäre abgelassen.

Die Standzeit des Wärmetauschers 3 betrug etwa ein Jahr.

Das Anfahren der Hochsiederkolonne kann mit Hilfe des Startverdampfers 18, der mit Heizdampf betrieben wird, geschehen oder auch mit Stickstoff erfolgen, der bei 31 auf der Saugseite des Brüdenverdichters 2 zugegeben wird, sich bei der Verdichtung aufgrund seines Adiabatenexponenten wesentlich stärker als EDC erhitzt und so lange über Leitung 28 im Kreis geführt wird, bis sich die richtige Brüdentemperatur am Kopf der Kolonne 1 eingestellt hat.

Vergleichsversuch

Man verfuhr analog Beispiel 5, jedoch ohne Thermokompression (Brüdenverdichtung). Zur Beheizung der Hochsiederkolonne 1 wurden beim Umlaufverdampfer 18 rund 18,5 t/h 16 bar-Dampf benötigt. Das gereinigte 1,2-Dichlorethan gleiche Zusammensetzung wie in Beispiel 5 gelangte mit etwa 116°C in den Economizer 9. Die Vorwärmung, Verdampfung, Spaltung und Aufarbeitung des Spaltgasgemisches wurde wie in Beispiel 5 beschrieben durchgeführt. Für die Beheizung der VCM-Kolonne waren 7 t/h 20 bar-Dampf erforderlich.

Das den Economizer 9 verlassende Rauchgas hatte eine Temperatur von etwa 315°C. Durch Wärmetausch im Abhitzekessel 12 bzw. Kesselspeisevorwärmer 13 kühlte sich das Rauchgas auf rund 177°C ab, bevor es in die Atmosphäre gelangte. Im Abhitzekessel wurden etwa 2 t/h 16 bar-Dampf erzeugt.

Die Vinylchloriderzeugung war gegenüber dem erfindungsgemässen Verfahren wesentlich unwirtschaftlicher, da rund 0,77 to Dampf pro Tonne erzeugtes Vinylchlorid mehr aufzuwenden waren. Der zusätzliche Stromverbrauch für die Thermokompression beim erfindungsgemässen Verfahren fiel demgegenüber kaum ins Gewicht. Ausserdem wurde durch das erfindungsgemässe Verfahren auch ein nicht unwesentlicher Beitrag für den Umweltschutz erzielt, da die Rauchgase mit wesentlich niedrigerer Temperatur in die Atmosphäre abgelassen wurden.

**Patentanspruch**

Verfahren zur Herstellung von Vinylchlorid durch thermische Spaltung von gereinigtem 1,2-Dichlorethan bei Temperaturen von 480°C bis 540°C unter einem Druck von 10 bis 36 bar absolut unter teilweiser Ausnutzung des Wärmeinhaltes der Rauchgase der Spaltofenbefeuerung zur Vorwärmung von flüssigem 1,2-Dichlorethan auf nahezu Siedetemperatur, unter Ausnutzung der Rauchgasabwärme zur Dampfgewinnung, Kühlen des Spaltgasgemisches in mehreren Stufen und Abtrennung des Chlorwasserstoffes aus dem Spaltgasgemisch in einer sog. HCl-Kolonne sowie Abtrennung von Vinylchlorid aus dem Spaltgasgemisch in einer sog. VCM-Kolonne, dadurch gekennzeichnet, dass man die Beheizung der Hochsiederkolonne zur Reinigung des 1,2-Dichlorethans, die mit einem Kopfdruck von 2 bis 3,1 bar absolut betrieben wird, durch Wärmetausch zwischen dem Sumpfprodukt und den auf 3,2 bis 6,2 bar absolut verdichteten Brüden bei einer Temperaturdifferenz von 8 bis 22°C vornimmt, die Sumpftemperatur durch entsprechenden Blasenabzug bei 120°C bis 135°C hält, das durch entsprechend der erhöhten Temperatur erniedrigte Verdampfungswärmen sich ergebende Wärmedefizit auf der wärmeabgebenden Seite des Wärmetauschers durch Entspannung entsprechender Mengen an Rücklauf kompensiert, den Teil des Kondensates aus dem Sumpfwärmetauscher, der als Rücklauf in die Kolonne zurückgeführt wird, im Wärmetausch mit Blasenprodukt aus der HCl-Kolonne, das zur VCM-Kolonne fliesst, auf die dem jeweiligen Kopfdruck entsprechende Sattdampftemperatur abkühlt, wobei sich das Blasenprodukt der HCl-Kolonne entsprechend anwärmt und sogar teilweise verdampft, den Rest des im Wärmetauscher anfallenden Brüdenkondensates, welches das gereinigte 1,2-Dichlorethan darstellt, ungekühlt mit einer Temperatur von 125°C bis 155°C direkt in den Economizer des thermischen Spaltofens pumpt und aus den Rauchgasen der Spaltofenbefeuerung bei einem Temperaturniveau von 350°C bis 550°C in einem Abhitzekessel durch Wärmetausch mit siedendem Wasser Mitteldruckdampf erzeugt, wobei das Kesselspeisewasser für den Abhitzekessel in einem dem Abhitzekessel nachgeschalteten Wärmetauscher durch Wärmetausch mit den Rauchgasen bei einem entsprechenden niedrigeren Temperaturniveau von 60 bis 120°C auf Siedetemperatur im Abhitzekessel vorgewärmt wird.

## Claim

Process for producing vinyl chloride by thermal cracking of purified 1,2-dichloroethane at temperatures of 480°C to 540°C under pressures of 10 to 36 bar absolute, with partial utilization of the heat content of the flue gases from the cracking furnace heating system for preheating liquid 1,2-dichloroethane almost to boiling point, with utilization of the flue gas waste heat for steam raising, cooling of the cracked gas mixture in several stages and separation of the hydrogen chloride from the cracked gas mixture in a so-called HCl column and separation of vinyl chloride from the cracked gas mixture in a so-called VCM column, characterized in that the high-boiler column for purifying the 1,2-dichloroethane, which is operated with a top pressure of 2 to 3.1 bar absolute, is heated by heat exchange between the bottom product and the vapour, compressed to 3.2 to 6.2 bar absolute, at a temperature difference of 8 to 22°C, in that the bottom temperature is kept at 120°C to 135°C by appropriate takeoff of bottoms, in that compensation is made for the heat deficit, which arises because of the reduced heat of vapourization corresponding to the increased temperature, on the heat output side of the heat exchanger by flash evaporation of appropriate quantities of reflux, in that the part of the condensate from the bottom heat exchanger which is returned to the column as reflux is cooled to the saturated vapour temperature corresponding to the particular top pressure in heat exchange with the bottom product from the HCl column which passes to the VCM column, the bottom product of the HCl column being correspondingly heated and even partially vapourized, in that the remainder of the vapour condensate produced in the heat exchanger, which represents the purified 1,2-dichloroethane, is pumped, uncooled, at a temperature of 125°C to 155°C directly into the economizer of the thermal cracking furnace and in that medium pressure steam is generated from the flue gases of the cracking furnace heating system at a temperature level of 350°C to 550°C in a waste heat boiler by heat exchange with boiling water, the boiler feed water for the waste heat boiler being preheated to boiling temperature in the waste heat boiler in a heat exchanger connected downstream of the waste heat boiler by heat exchange with the flue gases at a correspondingly lower temperature level of 60 to 120°C.

## Revendication

Procédé pour préparer du chlorure de vinyle par coupure de craquage thermique de dichloro-1,2 éthane purifié, à des températures de 480°C à 540°C sous une pression absolue de 10 à 36 bars, avec utilisation partielle de la chaleur contenue dans les gaz de fumée provenant du chauffage du four de craquage pour préchauffer le dichloro-1,2 éthane liquide jusqu'au voisinage de sa température d'ébullition, avec utilisation de la chaleur des gaz de fumée pour obtenir de la vapeur d'eau, refroidissement du mélange des gaz de craquage en plusieurs étapes et séparation du chlorure d'hydrogène de ce mélange des gaz de craquage dans ce que l'on appelle une colonne de HCl, ainsi que séparation du chlorure de vinyle du mélange des gaz de craquage dans ce que l'on appelle une colonne de chlorure de vinyle monomère, procédé caractérisé en ce qu'on effectue le chauffage de la colonne de séparation des produits à point élevé d'ébullition, pour la purification du dichloro-1,2 éthane, colonne que l'on fait fonctionner avec une pression absolue en tête de 2 à 3,1 bars, par échange de chaleur, à une différence de température de 8 à 22°C, entre le produit de pied de colonne et les vapeurs comprimées à une pression absolue de 3,2 à 6,2 bars; on maintient, par un prélèvement correpondant en pied ou cornue de colonne, la température du pied de colonne entre 120°C et 135°C; on compense, par détente de quantités correspondantes de reflux, le déficit de chaleur, dû à une diminution de chaleur d'évaporation correspondant à l'élévation de température, du côté de l'échangeur de chaleur cédant de la chaleur; on refroidit la partie du condensat provenant de l'échangeur de chaleur du produit de pied de colonne qui est recyclé comme reflux dans la colonne en échange de chaleur avec le produit de la cornue de la colonne de HCl, qui s'écoule vers la colonne de chlorure de vinyle monomère, pour amener cette partie à la température de vapeur saturée correspondant à la pression en tête de colonne, de sorte que le produit du pied ou de la cornue de la colonne de HCl s'échauffe de manière correspondante et est même partiellement évaporé; on pompe le reste du condensat des vapeurs, obtenu dans l'échangeur de chaleur, et qui constitue le dichloro-1,2 éthane purifié, non refroidi et ayant une température de 125°C à 155°C, directement dans l'économiseur du four de craquage thermique; et, à partir des gaz de fumée provenant du chauffage de ce four de craquage et ayant un niveau de températures de 350°C à 550°C, on produit dans une chaudière de récupération des chaleurs perdues, par échange de chaleur avec de l'eau bouillante, de la vapeur d'eau à pression moyenne, l'eau alimentant cette chaudière de récupération étant préchauffée, dans un échangeur de chaleur monté en aval de la chaudière, par échange de chaleur avec les gaz de fumée, d'un bas niveau correspondant de températures allant de 60°C à 120°C jusqu'à la température d'ébullition dans la chaudière de récupération des chaleurs perdues.

**Fig. 1**

0 180 925

Fig. 2